# EUROPEAN PATENT SPECIFICATION

(11) **EP 2 198 800 B1**
(45) Date of publication and mention of the grant of the patent: **08.08.2012**
(21) Application number: 09015583.9
(22) Date of filing: 16.12.2009
(51) Int. Cl.: A61B 18/20

(54) **Light-irradiating device for removing hair or suppressing revival and growth of hair**
Lichtbehandlungsvorrichtung zur Haarentfernung oder zur Unterdrückung des Haarwachstums
Dispositif lumineux pour l'épilation ou suppression de la pousse pileuse

(30) Priority: 22.12.2008 JP 2008326411
(43) Date of publication of application: 23.06.2010
(73) Proprietor: Panasonic Corporation, Osaka 571-8501 (JP)
(72) Inventor: Sueyoshi, Hidekazu, Kadoma-shi Osaka (JP); Wang, Wei, Kadoma-shi Osaka (JP); Ogawa, Hitoshi, Kadoma-shi Osaka (JP)
(74) Representative: Samson & Partner

(56) References cited:
- WO-A-03/043514
- WO-A-2007/007167
- GB-A- 2 110 839
- US-A- 1 923 531
- US-A- 4 356 538
- US-A- 4 460 942
- US-A- 4 533 984
- US-A- 6 111 359

## Description

### Field of the Invention

The present invention relates to a light-irradiating beauty care device and, more specifically, to a light-irradiating beauty care device provided with a light source and designed to treat a skin by irradiating the light generated from the light source on a living body surface, particularly on a skin surface.

### Background of the Invention

Conventionally, there have been available light-irradiating beauty care devices that treat a skin to remove hair or to suppress revival of hair by irradiating the light generated from a light source on a living body surface, particularly on a skin surface. As disclosed in Japanese Patent Laid-open Publication Nos. 2004-321401 and 2006-518612, the conventional light-irradiating beauty care devices include a hand-held main body of compact shape gripped by one hand when in use and a light irradiation unit provided in the main body.

In the beauty care devices of the patent documents cited above, the light irradiation unit is integrally formed with the main body and cannot be removed from the main body with ease. No consideration is given to replacement of the light irradiation unit, particularly replacement of a light source. Therefore, the light irradiation unit or the light source cannot be readily replaced even when the quantity of light is reduced due to the dislocation of the light source or the deterioration of the light irradiation unit caused by the dropping of the beauty care devices.

US 6,111,359 A, WO 2007/007167 A, US 4,356,538 A, US 4,533,984 A and US 1,923,531 A all disclose light-irradiating devices according to the preamble of claim 1 which are suitable for removing hair or suppressing revival and growth of hair.

### Summary of the Invention

In view of the above, the present invention provides a light-irradiating beauty care device capable of irradiating a stable quantity of light for a prolonged time period by preventing dislocation or deformation of a light source or deterioration thereof due to the falling shock applied to the beauty care device or due to the heat generated during light emission and by making it possible to correct the dislocation of the light source with ease.

While the invention is defined in independent claim 1, further aspects of the invention are set forth in the dependent claims, the drawings and the following description.

In accordance with an aspect of the present invention, there is provided a light-irradiating beauty care device including: a hand-held main body; and a light irradiation unit provided at one end of the main body for irradiating light on a living body surface, wherein the light irradiation unit includes a xenon tube as a light source for emitting the light, a reflector for reflecting the light emitted from the light source, a lens for passing the light therethrough, the reflector and the lens cooperating with each other to make uniform the quantity of the light emitted from the light source, a base for holding the reflector in place, a trigger for applying a trigger voltage to the light source and a casing for accommodating the light source, the reflector, the lens, the base and the trigger, and wherein the light irradiation unit is removably attached to the main body, the light source being fixed to the reflector, the reflector being held in place by the base and the lens.

With this configuration, the light irradiation unit can be removably attached to the main body, so that it is possible to easily replace the light irradiation unit which may suffer from over-time deterioration or deformation caused by the heat during the use of the light-irradiating beauty care device. The reflector carrying the light source is held in place by the lens and the base. This makes it possible to prevent dislocation of the light source with respect to the lens and the reflector.

The xenon tube may be fixed to the reflector by an elastic body and the reflector is attached to the base through another elastic body. In this case, it becomes possible to resiliently fix the xenon tube to the reflector while allowing the reflector to be resiliently held in the light irradiation unit by the base and the lens. Thus, it is possible for the elastic bodies to absorb and reduce the vibration and shock that would occur when the light-irradiating beauty care device is in use or when the light irradiation unit is attached to or removed from the main body. This makes it possible to prevent dislocation of the light source and, consequently, to stabilize the quantity of the light irradiated.

The casing may include an opening through which to dissipate heat. In this case, the heat generated in the light source is dissipated to the outside of the casing. This helps suppress temperature rise within the light irradiation unit during light emission. This also restrains the light irradiation unit and its component parts from being deteriorated or deformed by the heat generated during light emission, which assists in preventing reduction of the quantity of the light irradiated.

The reflector may be configured to make point-to-point contact with the lens. In this case, it is possible to prevent the heat generated in the light source from being transferred to the lens through the reflector. This restrains the light irradiation unit and its component parts from being deteriorated or deformed by the heat generated during light emission, which assists in preventing reduction of the quantity of the light irradiated.

With the light-irradiating beauty care device of the present invention, the light irradiation unit carrying the light source is removably attached to the main body. This makes it possible to easily remove the light source from the main body when the light source is deteriorated or out of order. Therefore, the light source suffering from over-time deterioration or trouble can be replaced with ease. The light source, the reflector and the lens heavily affected by the heat generated during light emission are integrated into a single unit. Even if the light source, the reflector and the lens are deteriorated or deformed during the long-term use of the light-irradiating beauty care device and even if the light source is dislocated resultantly, it is possible to correct the dislocation of the light source with ease by replacing the light irradiation unit. This makes it possible to irradiate a stable quantity of light for a prolonged period of time.

### Brief Description of the Drawings

Fig. 1 is an exploded perspective view showing a light irradiation unit employed in a light-irradiating beauty care device in accordance with an embodiment of the present invention.
Fig. 2A is a front view of the light irradiation unit and Fig. 2B is a section view taken along line 2B-2B in Fig. 2A.
Figs. 3A and 3B are front and side views showing the light-irradiating beauty care device.
Figs. 4A and 4B are section views taken along line 4A-4A in Fig. 3A and line 4B-4B in Fig. 3B, respectively.
Fig. 5 is a perspective view of the beauty care device shown in Figs. 4A and 4B, with a cover removed for clarity.
Fig. 6A is a front view showing the cover removed from the beauty care device and Fig. 6B is a section view taken along line 6B-6B in Fig. 6A.
Fig. 7 is a perspective view showing a float block employed in the light-irradiating beauty care device.

### Detailed Description of the Preferred Embodiments

Hereinafter, one embodiment of the present invention will be described with reference to the accompanying drawings.

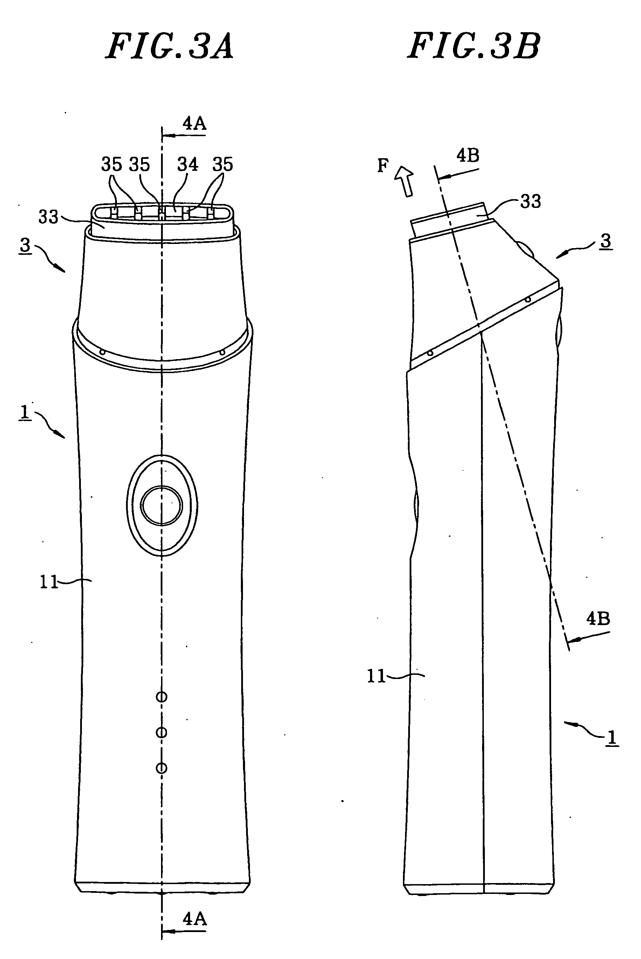

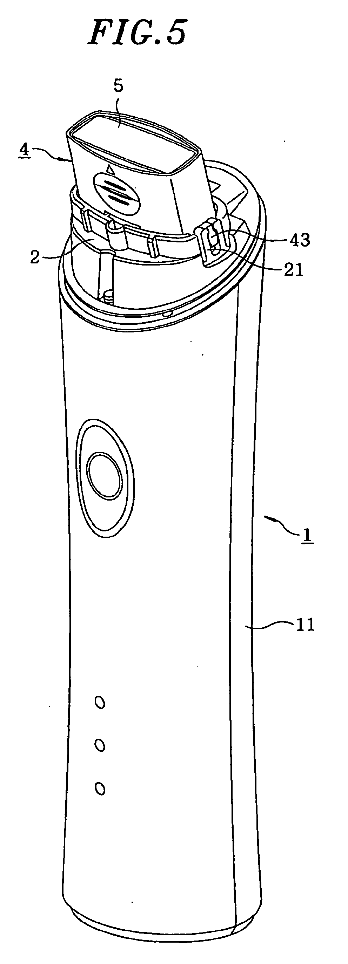

A light-irradiating beauty care device 1 in accordance with the embodiment of the present invention is configured to irradiate light on a living body surface, particularly on a skin surface, to perform beauty care such as light-aided hair removal by which to remove hair from the living body surface or light-aided hair growth suppression by which to suppress revival and growth of hair on the living body surface. Referring to Figs. 3A through 5, the beauty care device 1 includes a main body 11 which can be held with one hand, a light irradiation unit 4 removably attached to one end of the main body 11, and a cover 3 removably attached to the main body 11 for covering the periphery of the light irradiation unit 4.

The main body 11 of the light-irradiating beauty care device 1 includes a built-in power source 12, e.g., a battery, and a control circuit 13 for controlling the irradiation of light, both of which are arranged inside the main body 11. At the light-irradiating end portion 2 of the main body 11 to which the light irradiation unit 4 is attached, there are provided unit holder portions 21, i.e., two substantially U-shaped claws, for removably holding the light irradiation unit 4, a body side connector 22 protruding from the light-irradiating end portion 2 to electrically interconnect the main body 11 and the light irradiation unit 4, and a body side locking portion 23 for removably locking the cover 3.

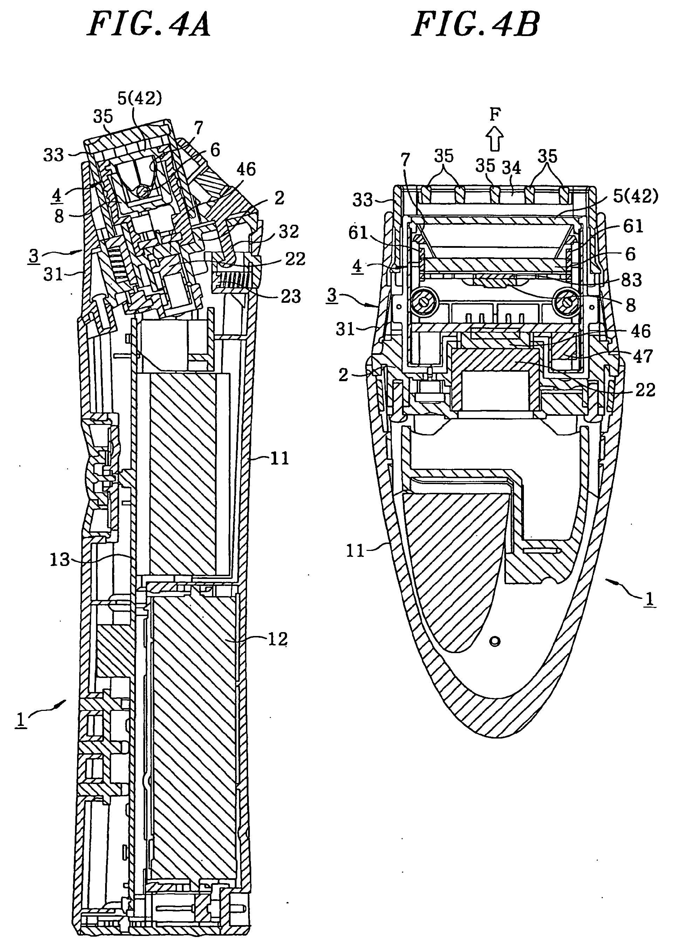

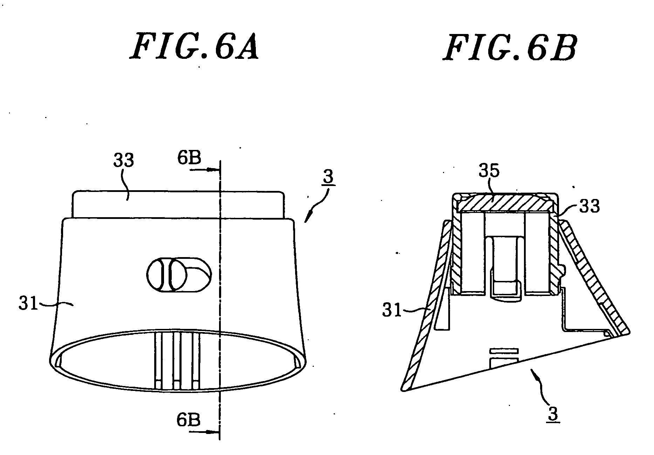

As can be seen in Figs. 4A, 4B, 6A and 6B, the cover 3 includes a cover member 31 for covering the light-irradiating end portion 2 and the light irradiation unit 4, a cover side locking portion 32 engageable with the body side locking portion 23 for removably attaching the cover member 31 to the main body 11, and a tubular float block 33 provided with a discharge mouth 34 through which to discharge the light of the light irradiation unit 4 to the outside.

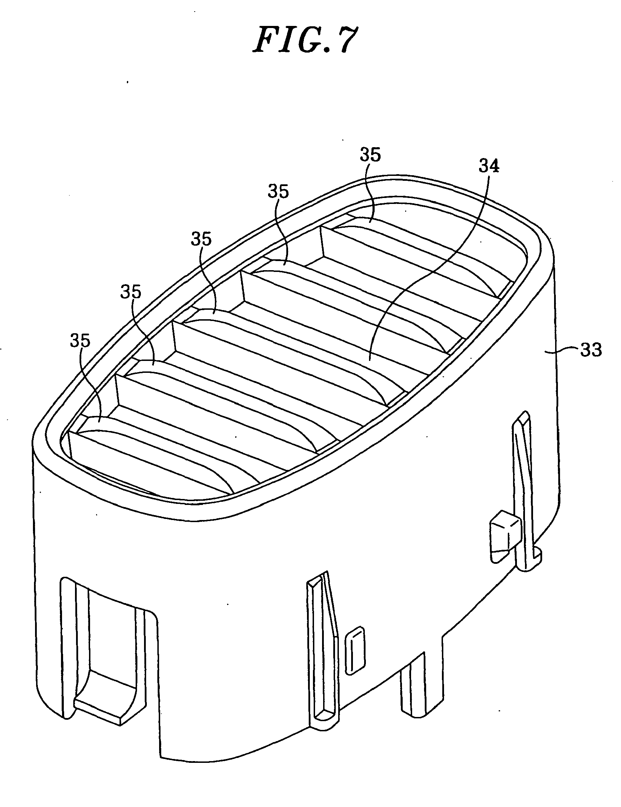

As illustrated in Figs. 6A, 6B and 7, the float block 33 is attached to the cover member 31 so that it can make floating movement in the light irradiation direction F, namely in the direction perpendicular to the discharge mouth 34. If the light irradiation unit 4 is energized with the discharge mouth 34 pressed against the living body surface, the light is irradiated on the living body surface through the discharge mouth 34. The float block 33 is made of a light interrupting material. The light irradiation unit 4 is arranged inside the float block 33, which prevents the light of the light irradiation unit 4 from being leaked through other portions than the discharge mouth 34.

A grid 35 formed of optical members is provided in the discharge mouth 34. When the discharge mouth 34 of the float block 33 is pressed against the living body surface to perform light irradiation, the grid 35 restrains the living body surface from coming into the float block 33, thereby keeping the living body surface out of contact with the light irradiation unit 4. The optical members forming the grid 35 can transmit the light uniformly emitted from the light irradiation unit 4 without substantially changing the intensity and direction thereof. This eliminates the possibility that the quantity of light is made non-uniform by the grid 35.

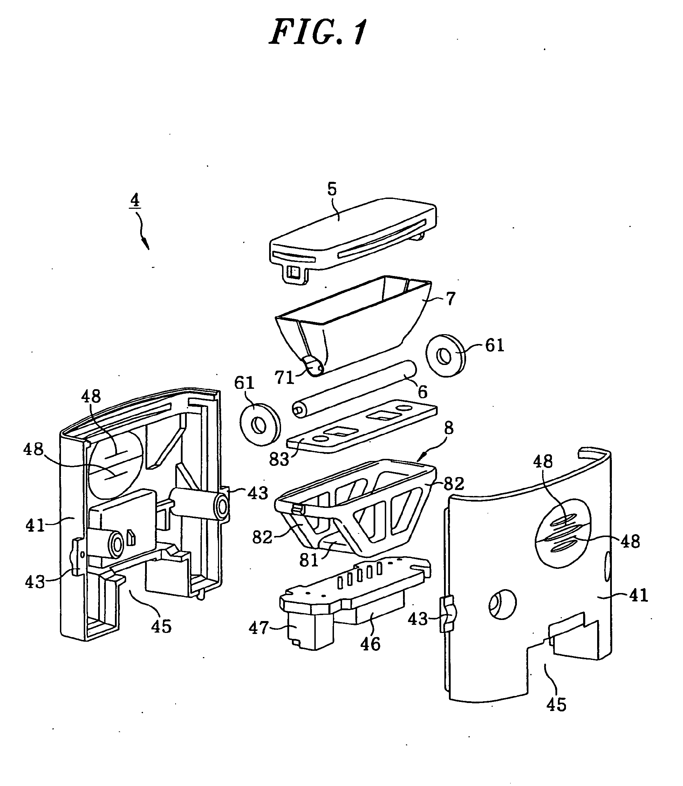

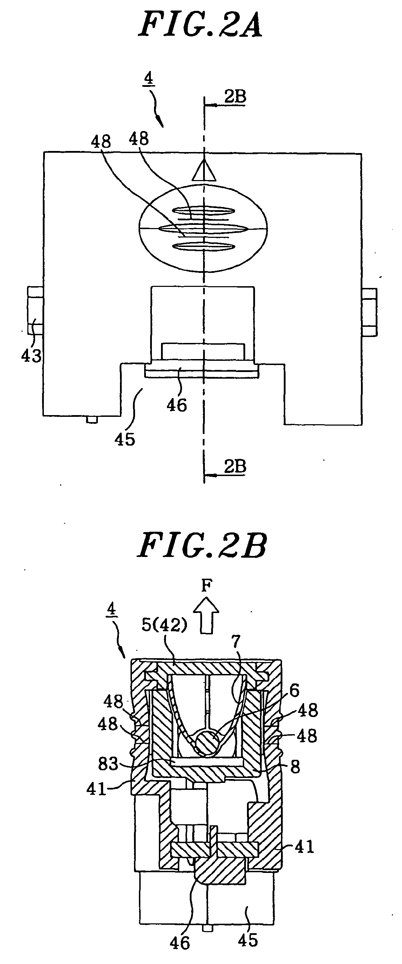

The light irradiation unit 4 has a generally rectangular box-shaped casing provided at one end with an irradiation mouth 42 communicating with the discharge mouth 34. The light of the light irradiation unit 4 is irradiated to the outside through the irradiation mouth 42. As shown in Figs. 1, 2A and 2B, the box-shaped casing is longitudinally halved into two case members 41. A lens 5 as a lid is fitted to the irradiation mouth 42.

Installation lugs 43 are formed at the longitudinally opposite side surfaces of the box-shaped casing. The installation lugs 43 are engageable with the U-shaped claws provided in the light-irradiating end portion 2 of the main body 11. A recess portion 45 is formed at the generally central portion of the bottom end opposite from the irradiation mouth 42. Within the recess portion 45, there is provided an irradiation side connector 46 electrically connectable to the body side connector 22 when the light irradiation unit 4 is attached to the main body 11.

The light irradiation unit 4 comes into the mounting position if the recess portion 45 of the light irradiation unit 4 is aligned with the body side connector 22 of the light-irradiating end portion 2 of the main body 11 by gripping the transversely opposite side surfaces of the case members 41 generally perpendicular to the longitudinally opposite side surfaces provided with the installation lugs 43. Then, the light irradiation unit 4 is pushed in the opposite direction from the light irradiation direction F so that the installation lugs 43 can be fitted to the U-shaped claws of the main body 11. Consequently, the installation lugs 43 are retained in place by the claws whereby the light irradiation unit 4 is mounted to the main body 11.

If the light irradiation unit 4 mounted to the main body 11 is pulled in the light irradiation direction F by gripping the transversely opposite side surfaces of the case members 41, the installation lugs 43 are drawn from the claws so that the light irradiation unit 4 can be removed from the main body 11.

A plurality of slits 48 as openings, which allow the inside and the outside of the light irradiation unit 4, are formed on the transversely opposite side surfaces of the case members 41 to extend in the longitudinal direction. The heat generated inside the light irradiation unit 4 is dissipated to the outside through the slits 48.

Within the box-shaped casing covered with the lens 5, there are arranged, for example, a xenon tube 6 of tubular bulb shape extending in the longitudinal direction of the box-shaped casing and serving as a light source, a reflector 7 for reflecting the light coming from the light source toward the irradiation mouth 42, a base 8 for holding the reflector 7 in cooperation with the lens 5 in a state that an elastic member 83 is interposed between the reflector 7 and the base 8, and a trigger 47 for applying a trigger voltage to the xenon tube 6 and causing the xenon tube 6 to emit light in response to a signal supplied from the light-irradiating connector 46.

The reflector 7 has a cup shape with a generally U-shape section, and includes a generally rectangular opening formed near the irradiation mouth 42 and a reflection surface arranged at the inner side thereof. The reflector 7 makes uniform the light emitted from the light source in cooperation with the lens 5. Then, the light is irradiated on the living body surface through the discharge mouth 34. In the cup-shaped bottom portion lying opposite from the opening of the reflector 7, insertion holes 71 through which to insert the xenon tube 6 are formed at the opposite longitudinal end sides. The light source is positioned inside the reflector 7 by inserting the xenon tube 6 through the insertion holes 71.

Elastic bodies 61, e.g., rubber plates of generally doughnut shape, are fitted to the axial opposite ends of the xenon tube 6. The elastic bodies 61 face toward each other and make contact with the side surfaces of the reflector 7 around the insertion holes 71, thereby pinching the reflector 7 at the opposite ends thereof and elastically fixing the xenon tube 6 to the reflector 7.

The base 8 includes a seat portion 81 for supporting the bottom portion of the reflector 7 and a side portion 82 protruding from the seat portion 81 to hold the outer surface of the reflector 7 lying at the opposite side of the reflection surface. The base 8 is provided with a plurality of openings through which to dissipate the heat generated by the operation of the light source and the reflector 7. An elastic member 83, e.g., a rubber plate of generally flat shape, is interposed between the bottom portion of the reflector 7 and the base 8. More specifically, the bottom portion of the reflector 7 and the base 8 make contact with the opposite surfaces of the elastic member 83, respectively.

The side portion 82 of the base 8 includes retainer portions engageable with the peripheral portion of the lens 5. The base 8 and the lens 5 are coupled together by the retainer portions. Thus, the four end sides of the reflector 7 defining the opening thereof make contact with the lens 5. The reflector 7 is pinched between the base 8 and the lens 5 to thereby compress the elastic member 83 lying between the base 8 and the reflector 7. Consequently, the lens 5 and the base 8 resiliently hold the reflector 7 in place.

Although not particularly shown in the drawings, a plurality of protrusions is formed on the end sides of the reflector 7 making contact with the lens 5, in such a manner that they do not cover the opening of the reflector 7. The end sides of the reflector 7 resiliently held in place are not direct contacted with the lens 5. Instead, the protrusions of the reflector 7 make point-to-point contact with the lens 5. This prevents the heat generated during light emission from being transferred to the lens 5 through the reflector 7. The protrusions are formed to extend in the light irradiation direction F, e.g., at six points, namely at four corners joining the respective end sides of the reflector 7 and at two center points of the longer end sides of the reflector 7. The protrusions have a diameter substantially equal to the thickness of the reflector 7, namely, the dimension between the reflection surface and the outer surface of the reflector 7.

With the light-irradiating beauty care device described above, the light irradiation unit 4 with a light source is removably attached to the main body 11 in such a manner that does not require the use of a screw driver or other tools in attaching and removing the light irradiation unit 4. This eliminates the need to use a tool in the attachment and removal process and, therefore, makes it easy to remove the light irradiation unit 4 from the main body 11 at the time of occurrence of deterioration or trouble. Therefore, the light irradiation unit 4 suffering from over-time deterioration or trouble can be replaced with ease, which makes it possible to irradiate a stable quantity of light for a long time period. The term "long time period" used herein means the time period required in repeatedly using the light-irradiating beauty care device day by day and not the time period taken in using the beauty care device once to perform light irradiation several times.

The light source, the reflector 7 and the lens 5, all of which are easy to grow hot by the heat generated during light irradiation, are integrally formed into the light irradiation unit 4. Even if the light source, the reflector 7 and the lens 5 are deteriorated or deformed during the long-term use of the light-irradiating beauty care device and even if the light source is dislocated resultantly, it is possible to correct the dislocation of the light source with ease by replacing the light irradiation unit 4. This makes it possible to irradiate a stable quantity of light for a prolonged period of time.

The light source is resiliently fixed to the reflector 7 by using the elastic bodies 61. Furthermore, the reflector 7 is resiliently held between the lens 5 and the base 8 through the elastic body 83. This prevents the light source from being misaligned with the lens 5 and the reflector 7. This also makes it possible for the elastic bodies 61 and 83 to absorb and reduce the vibration and shock that would occur when the discharge mouth 34 of the float block 33 is pressed against the living body surface to perform light irradiation or when the light irradiation unit 4 is attached to or removed from the main body 11. Therefore, it is possible to restrain the light source and the reflector 7 from coming out of position. This makes it possible to prevent dislocation of the light source and, consequently, to prevent the light irradiated through the irradiation mouth 42 from becoming non-uniform. As a result, it becomes possible to irradiate a stable quantity of light for an extended period of time.

The casing is provided with the plurality of slits 48 through which to dissipate the heat generated in the light irradiation unit 4 during light irradiation to the outside. This helps suppress temperature rise within the light irradiation unit 4. The reflector 7 and the lens 5 are configured to make point-to-point contact with each other, which prevents the heat generated in the reflector 7 during light emission from being transferred to the lens 5. This restrains the light irradiation unit 4 from being deteriorated or deformed by the heat generated during light emission, which assists in preventing dislocation of the light source and enhancing durability of the light irradiation unit 4. Accordingly, it becomes possible to irradiate a stable quantity of light for an extended period of time.

Although the elastic bodies 61 and 82 are independently formed in the present embodiment, this does not limit the present invention but is nothing more than one preferred example. The elastic bodies 61 and 82 may be formed into a single unit or modified to other designs insofar as the advantageous effects of the present invention can be attained.

While the invention has been shown and described with respect to the embodiments, it will be understood by those skilled in the art that various changes and modifications may be made without departing from the scope of the invention as defined in the following claims.

## Claims

1. A light-irradiating device (1) for removing hair or suppressing revival and growth of hair comprising:
a hand-held main body (11); and
a light irradiation unit (4) provided at one end of the main body (11) for irradiating light on a living body surface,
wherein the light irradiation unit (4) includes a light source for emitting the light, a reflector (7) for reflecting the light emitted from the light source, a lens (5) for passing the light therethrough, the reflector (7) and the lens (5) cooperating with each other to make uniform the quantity of the light emitted from the light source, a base (8) for holding the reflector (7) in place, a trigger for applying a trigger voltage to the light source and a casing for accommodating the light source, the reflector (7), the lens (5), the base (8) and the trigger (47),
wherein the light irradiation unit (4) is removably attached to the main body (11), the light source being fixed to the reflector (7), the reflector (7) being held in place by the base (8) and the lens (5),
**characterized in that** the light source is fixed to the reflector (7) by an elastic body (61) and the reflector (7) is attached to the base (8) through another elastic body (83).

2. The device of claim 1, wherein the casing includes an opening for dissipating heat therethrough.

3. The device of claim 1 or 2, wherein the reflector (7) is configured to make point-to-point contact with the lens (5),
wherein a plurality of protrusions is formed on end sides of the reflector (7) making contact with the lens (5) in such a manner that they do not cover the opening of the reflector (7),
wherein the end sides of the reflector (7) resiliently held in place are not in direct contat with the lens (5),
wherein the protrusions of the reflector (7) make point-to-point contact with the lens (5), and
wherein the protrusions are formed to extend in a light irradiation direction.

4. The device of any one of claims 1 to 3, wherein the light source is a xenon tube (6).

## Patentansprüche

1. Lichtstbestrahlungsvorrichtung (1) zur Haarentfernung oder Unterdrückung einer Belebung und Wachstum von Haar umfassend:
einen handgehaltenen Hauptkörper (11); und
eine Lichtbestrahlungseinheit (4), die an einem Ende von dem Hauptkörper (11) zum Strahlen von Licht auf eine lebendige Körperoberfläche vor gesehen ist,
wobei die Lichtbestrahlungseinheit (4) eine Lichtquelle umfasst, zum Emittieren des Lichts, einen Reflektor (7) zum Reflektieren des Lichts, das von der Lichtquelle emittiert wurde, eine Linse (5) zum Passieren des Lichtes dort durch, wobei der Reflektor (7) und die Linse (5) derart miteinander zusammenwirken, um die Lichtmenge, die von der Lichtquelle emittiert wurde, gleichmäßig zu machen, eine Basis (8) zum Halten des Reflektors (7) an seiner Stelle, einen Auslöser zum Anwenden einer Auslösespannung auf die Lichtquelle und ein Gehäuse zum Aufnehmen der Lichtquelle, des Reflektors (7), der Linse (5), der Basis (8) und des Auslösers (47),
wobei die Lichtbestrahlungseinheit (4) an dem Hauptkörper (11) entfernbar befestigt ist, wobei die Lichtquelle an den Reflektor (7) fixiert ist, wobei der Reflektor (7) über die Basis (8) und die Linse (5) an seiner Stelle gehalten wird,
**dadurch gekennzeichnet, dass** die Lichtquelle an dem Reflektor (7) über einen elastischen Körper (61) fixiert ist und der Reflektor (7) an der Basis (8) über einen anderen elastischen Körper (83) befestigt ist.

2. Vorrichtung gemäß Anspruch 1, bei der das Gehäuse eine Öffnung zum Ableiten von Wärme dadurch umfasst.

3. Vorrichtung gemäß Anspruch 1 oder 2, bei der der Reflektor (7) ausgelegt ist, um einen Punkt-zu-Punkt Kontakt mit der Linse (5) zu haben,
wobei mehrere Vorsprünge an Endseiten von dem Reflektor (7) ausgebildet sind, die mit der Linse (5) derart in Kontakt treten, dass sie nicht die Öffnung des Reflektors (7) abdecken,
wobei die Endseiten des Reflektors (7) die elastisch an ihrer Stelle gehalten sind, nicht in einem direkten Kontakt mit der Linse (5) haben,
wobei die Vorsprünge des Reflektors (7) einen Punkt-zu-Punkt Kontakt mit der Linse (5) haben, und
wobei die Vorsprünge ausgebildet sind, um sich in eine Lichtabstrahlrichtung zu erstrecken.

4. Vorrichtung gemäß einem der Ansprüche 1 bis 3, bei der die Lichtquelle eine Xenonröhre (6) ist.

## Revendications

1. Dispositif d'irradiation à la lumière (1) destiné à éliminer des poils ou à supprimer la renaissance et la croissance de poils, comprenant :
un corps principal tenu dans la main (11) ; et
une unité d'irradiation à la lumière (4) disposée au niveau d'une extrémité du corps principal (11) destinée à irradier de la lumière sur une surface d'un corps vivant ;
dans lequel l'unité d'irradiation à la lumière (4) comprend une source de lumière destinée à émettre la lumière, un réflecteur (7) destiné à réfléchir la lumière émise par la source de lumière, une lentille (5) destinée à être traversée par la lumière, le réflecteur (7) et la lentille (5) coopérant l'un avec l'autre de façon à rendre uniforme la quantité de la lumière émise par la source de lumière, une base (8) destinée à tenir le réflecteur (7) en place, un dispositif de déclenchement destiné à appliquer une tension de déclenchement à la source de lumière et un boîtier destiné à recevoir la source de lumière, le réflecteur (7), la lentille (5), la base (8) et le dispositif de déclenchement (47) ;
dans lequel l'unité d'irradiation à la lumière (4) est fixée de manière amovible au corps principal (11), la source de lumière étant fixée au réflecteur (7), le réflecteur (7) étant tenu en place par la base (8) et par la lentille (5) ;
**caractérisé en ce que** la source de lumière est fixée au réflecteur (7) par un corps élastique (61) et le réflecteur (7) est fixé à la base (8) par un autre corps élastique (83).

2. Dispositif selon la revendication 1, dans lequel le boîtier comprend une ouverture destinée à dissiper la chaleur à travers celle-ci.

3. Dispositif selon la revendication 1 ou la revendication 2, dans lequel le réflecteur (7) est configuré de façon à établir un contact point à point avec la lentille (5) ;
dans lequel une pluralité de saillies sont formées sur des côtés d'extrémité du réflecteur (7) établissant un contact avec la lentille (5) de telle façon qu'elles ne couvrent pas l'ouverture du réflecteur (7) ;
dans lequel les côtés d'extrémité du réflecteur (7) tenus en place de manière élastique ne sont pas en contact direct avec la lentille (5) ;
dans lequel les saillies du réflecteur (7) établissent un contact point à point avec la lentille (5) ; et
dans lequel les saillies sont formées de façon à s'étendre dans la direction d'irradiation de la lumière.

4. Dispositif selon l'une quelconque des revendications 1 à 3, dans lequel la source de lumière est un tube au xénon (6).
